# EUROPEAN PATENT APPLICATION

(11) **EP 4 737 401 A1**
(43) Date of publication of application: **06.05.2026**
(21) Application number: 25211500.1
(22) Date of filing: 27.10.2025
(51) Int. Cl.: C02F 1/32, F21V 5/00, G02B 19/00, A61L 2/10, A61L 9/20, F21V 5/04, H10H 20/00

(54) **LIGHT SOURCE MODULE AND ULTRAVIOLET LIGHT FLUID PROCESSING DEVICE**

(30) Priority: 01.11.2024 JP 2024193222; 30.05.2025 JP 2025091323
(71) Applicant: NICHIA CORPORATION, Tokushima 774-8601 (JP)
(72) Inventor: SANO, Hiroki, Anan-shi, Tokushima, 774-8601 (JP)
(74) Representative: Betten & Resch

(57) **Abstract**

A light source module includes a substrate, light sources positioned on an upper surface of the substrate, and an optical member including light control parts and positioned above the substrate such that the light sources are arranged between the substrate and the optical member.

## Description

### BACKGROUND

### 1. Field of the Invention

The present disclosure relates to a light source module and an ultraviolet light fluid processing device.

### 2. Description of the Related Art

There are lighting devices with multiple light emitting parts. For example, Unexamined Japanese Patent Application Publication No. 2016-170912 discloses a lighting device that includes first light emitting parts composed of multiple first light emitting elements connected in series, and second light emitting parts composed of multiple second light emitting elements connected in series and emitting light in a different color from that of the first light emitting parts, and, in this lighting device, the light distribution angle of emitted light changes according to switching of the color of emitted light by turning on the first light emitting parts and the second light emitting parts selectively.

### SUMMARY

According to an embodiment of the present disclosure, a light source module includes: a substrate having a center region and an outer region located outward of the center region; light sources positioned on an upper surface of the substrate and including a plurality of first light emitting parts; and an optical member positioned above the substrate such that the light sources are located between the substrate and the optical member, the optical member including light control parts that include a plurality of first light control parts. Each of the plurality of first light control parts overlaps with a respective one of the plurality of first light emitting parts in a top view so as to constitute a plurality of first stacked bodies positioned in the outer region. In each of the plurality of first stacked bodies, the first light control part includes: a first incident surface that is planar in shape and that faces the first light emitting part; and a first emission surface that is curved to be convex in a direction away from the first incident surface. In each of the plurality of first stacked bodies, in any cross section along a first axis that passes through a center of the first emission surface in the top view and that is perpendicular to the first incident surface, both sides of the first emission surface with respect to the first axis have a same curvature. In at least one of the plurality of first stacked bodies, an optical axis of the first light control part is non-parallel to the first axis.

### BRIEF DESCRIPTION OF THE DRAWINGS

FIG. 1A is a perspective view showing an example of a light source module according to a first embodiment of the present disclosure;
FIG. 1B is a diagram showing a cross section taken along the line IB-IB in FIG. 1A;
FIG. 2 is a partial top view (1) showing an example of the light source module of the first embodiment;
FIG. 3 is a partial top view of the light source module shown in FIG. 2 without the optical member;
FIG. 4 is a partial top view (2) showing an example of the light source module of the first embodiment;
FIG. 5 is a schematic diagram showing an enlarged view of the part labeled "A1" in FIG. 4;
FIG. 6 is a schematic diagram showing a cross section of a first stacked body S1, taken along the line VI-VI in FIG. 5;
FIG. 7 is a cross-sectional view (1) showing examples of second stacked bodies;
FIG. 8 is a cross-sectional view (2) showing examples of second stacked bodies;
FIG. 9 is a partial top view (3) showing an example of the light source module of the first embodiment;
FIG. 10 is a schematic diagram for explaining the direction in which light is emitted from the light source module of the first embodiment;
FIG. 11 is a partial top view (1) showing an example of a light source module according to a first modified example of the first embodiment;
FIG. 12 is a schematic diagram showing an enlarged view of the part labeled "A2" in FIG. 11;
FIG. 13 is a schematic diagram showing a cross section of a third stacked body S3, taken along the line XIII-XIII in FIG. 12;
FIG. 14 is a partial top view (2) showing an example of a light source module according to a first modified example of the first embodiment;
FIG. 15 is a schematic diagram for explaining the direction in which light is emitted from the light source module of the first modified example of the first embodiment;
FIG. 16 is a partial top view showing an example of a light source module according to a second modified example of the first embodiment;
FIG. 17 is a schematic diagram for explaining the direction in which light is emitted from the light source module of the second modified example of the first embodiment;
FIG. 18 is a partial top view showing an example of a light source module according to a third modified example of the first embodiment;
FIG. 19 is a schematic diagram for explaining the direction in which light is emitted from the light source module of the third modified example of the first embodiment;
FIG. 20 is a perspective view showing an example of an optical member for use in a light source module according to a fourth modified example of the first embodiment;
FIG. 21 is a perspective view showing an example of an ultraviolet light fluid processing device according to a second embodiment of the present disclosure;
FIG. 22 is a cross-sectional view taken along the line XXII-XXII in FIG. 21;
FIG. 23 is a perspective view showing an example of an ultraviolet light fluid processing device according to a first modified example of the second embodiment; and
FIG. 24 is a cross-sectional view taken along the line XXIV-XXIV in FIG. 23.

### DETAILED DESCRIPTION

The present disclosure aims to provide a light source module that can easily irradiate a specific region with light.

According to one embodiment of the present disclosure, a light source module can light a specific region with ease.

Hereinafter, certain embodiments of the present disclosure will be described with reference to the accompanying drawings. In the following description, terms that indicate specific directions or positions (e.g., "upper," "lower," etc.) are used where appropriate. However, these terms are used only for the purpose of facilitating understanding of the present disclosure with reference to the accompanying drawings, and the meaning of these terms does not limit the technical scope of the present invention. Also, parts that appear in multiple drawings with the same reference numerals indicate the same or equivalent parts or members.

Embodiments and examples shown hereinafter are examples of light source modules and related components for giving a concrete form to the technical idea of the present disclosure, and is not intended to limit the technical scope of the present invention to description herein. Also, unless otherwise specified, the dimensions, materials, shapes, relative positions, etc. of the components described below are intended to be illustrative and are not intended to limit the technical scope of the present disclosure. The details described in relationship to one embodiment are applicable to other embodiments, modified examples, examples, etc. The size, positional relationship, etc. of the members shown in the drawings may be exaggerated for ease of explanation. Furthermore, in order to avoid the drawings from becoming excessively complicated, schematic diagrams in which certain elements are omitted may be used.

### <First Embodiment>

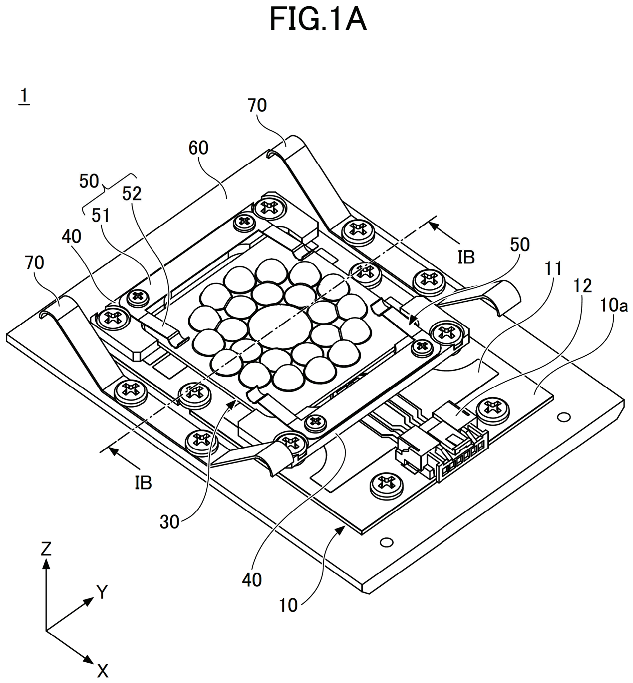

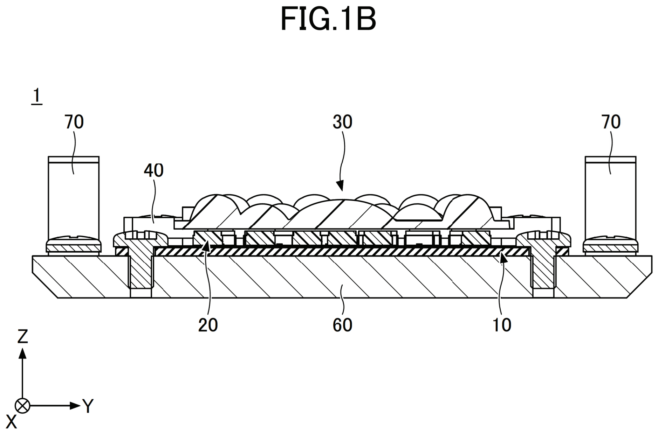

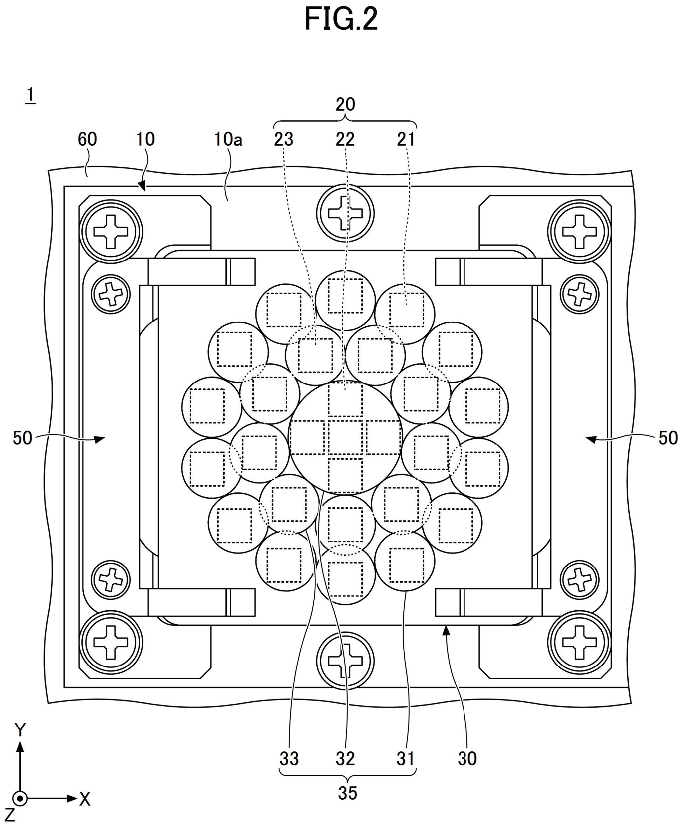

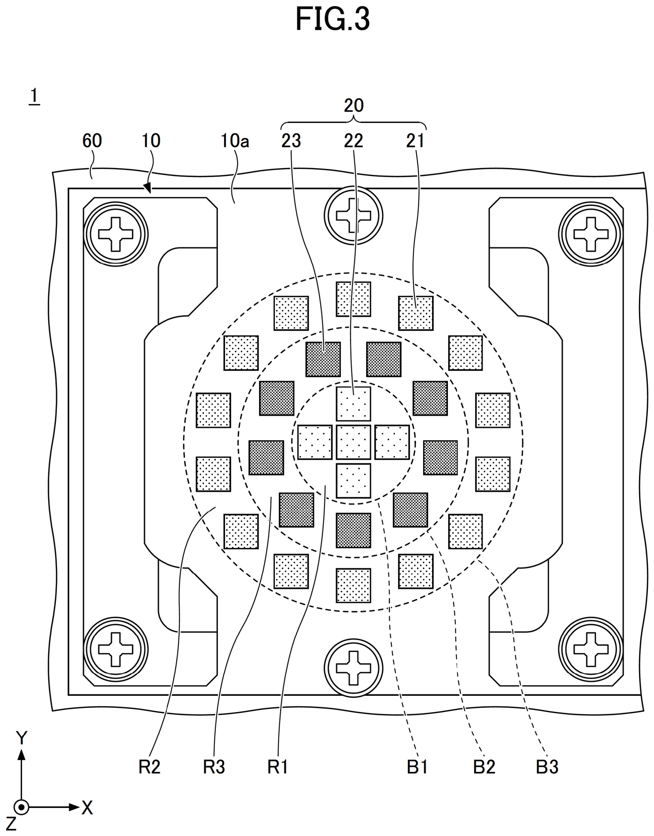

FIG. 1A is a perspective view showing an example of a light source module according to a first embodiment of the present disclosure. FIG. 1B is a diagram showing a cross section taken along the line IB-IB in FIG. 1A. FIG. 2 is a partial top view (1) showing an example of the light source module of the first embodiment. FIG. 3 is a partial top view in which the optical member is removed from the light source module shown in FIG. 2.

In these drawings, an X-axis, a Y-axis, and a Z-axis that are mutually perpendicular are shown for reference. Directions that are parallel to the X-axis will be hereinafter referred to as "first direction(s) X," directions that are parallel to the Y-axis will be hereinafter referred to as "second direction(s) Y," and directions that are parallel to the Z-axis will be hereinafter referred to as "third direction(s) Z." For example, in the first directions X, the direction in which the arrow points is the "+X direction," and the direction opposite to the +X direction is the "-X direction." In the second directions Y, the direction in which the arrow points is the "+Y direction," and the direction opposite to the +Y direction is the "-Y direction." In the third directions Z, the direction in which the arrow points is the "+Z direction," and the direction opposite to the +Z direction is the "-Z direction." However, these directions does not limit the orientation of the light source module upon use, and the light source module may be positioned and oriented in any appropriate direction.

As shown in FIG. 1A to FIG. 3, the light source module 1 includes a substrate 10, light sources 20, an optical member 30, pedestal parts 40, fixing members 50, a holding member 60, and a spring member 70. The light source module 1 does not necessarily include the pedestal parts 40, the fixing members 50, the holding member 60, and the spring member 70.

The substrate 10 is, for example, rectangular in a top view. In the illustrated examples, the upper surface 10a and the lower surface of the substrate 10 are flat surfaces, and the long sides of the upper surface 10a are parallel to the first direction X, and the short sides are parallel to the second direction Y. Also, the normal to the upper surface 10a is parallel to the third direction Z. However, the substrate 10 is not limited to be shaped rectangular in a top view. The substrate 10 has a predetermined pattern of a conductive member 11. A connector 12 that is electrically connected to the conductive member 11 may or may not be arranged on the upper surface 10a of the substrate 10. Unless otherwise specified, "top view" used herein means a view of the light source module 1 in the direction normal to the upper surface 10a of the substrate 10.

The light sources 20 are positioned on the upper surface 10a of the substrate 10. The optical member 30 is positioned above the substrate 10, with the light sources 20 between the optical member 30 and the substrate 10 in the Z direction. The optical member 30 may or may not be in contact with the light sources 20. The light sources 20 and the optical member 30 will be described in detail later.

In the illustrated examples, a pair of pedestal parts 40 are fixed onto the substrate 10, and the optical member 30 is held by the pedestal parts 40. To be more specific, a pair of fixing members 50 are provided on the respective upper surfaces of the pedestal parts 40 such that, in a top view, the fixing members 50 face each other with the optical member 30 disposed therebetween.

The fixing members 50 are, for example, metal plates. Each fixing members 50 includes, for example, a fixing part 51 and spring parts 52. Each fixing part 51 extends in the short-side direction (Y direction) of the upper surface 10a of the substrate 10 and is fixed to the pedestal part 40 below it. The spring parts 52 extend from both ends of the fixing part 51, onto the upper surface of the optical member 30. Each fixing part 51 can be fixed to the upper surface of the pedestal part 40 by, for example, screwing. The fixing members 50 press the optical member 30 against the substrate 10, so that the optical member 30 is held by the pedestal parts 40.

The substrate 10, on which the light sources 20 and the optical member 30 are arranged, can be fixed, for example, on a plate-shaped holding member 60. The substrate 10 can be fixed on the upper surface of the holding member 60, for example, by screwing, by an adhesive, etc.

Two spring members 70, extending in the long-side direction (X direction) of the upper surface 10a of the substrate 10, are positioned on the holding member 60. The spring members 70 can be positioned such that they face each other with the substrate 10 therebetween in a top view. The spring members 70 are, for example, flat springs made of metal. The spring members 70 can be fixed to the upper surface of the holding member 60 by, for example, screwing. The number of spring members 70 is not limited to two and may be any number greater than or equal to one. The spring members 70 are not limited to be in the shape shown in FIG. 1A and FIG. 1B and may be in any appropriate shape. The spring members 70 can be used when fixing the light source module 1 in a predetermined space.

The light source module 1 may have a jack mechanism instead of the spring members 70. This jack mechanism may be, for example, configured such that, for example, rotation of a spring member causes a support member to move upward and downward relative to the upper surface of the holding member 60. For example, the light source module 1 can be fixed in a predetermined space by inserting the light source module 1 in a predetermined space in a state in which the support member has been lowered and then rotating the spring member to raise the support member.

As shown in FIG. 3, the substrate 10 at least includes a center region R1 and an outer region R2 located outward of the center region R1, which are regions where the light sources 20 are positioned. The center region R1 and the outer region R2 are regions on the upper surface 10a of the substrate 10.

In the example illustrated in FIG. 3, the substrate 10 further includes a middle region R3 located between the center region R1 and the outer region R2. The center region R1 is located closer to the center than is a boundary B1. The outer region R2 is located between a boundary B2 and a boundary B3. The middle region R3 is located between the boundary B1 and the boundary B2. The boundary B1 is located inward of the boundary B2. The boundary B2 is located inward of the boundary B3 and outward of the boundary B1. In FIG. 3, the boundary B1, the boundary B2, and the boundary B3 are shown with dashed lines, and these lines are virtual lines.

The light sources 20 include a plurality of first light emitting parts 21. In the example shown in FIG. 1A to FIG. 3, the light sources 20 further include one or more second light emitting parts 22 and a plurality of third light emitting parts 23. Referring to FIG. 3, for ease of understanding, all the first light emitting parts 21 are shown in a dot pattern of a unique density, all the second light emitting parts 22 are shown in a dot pattern of another unique density, and all the third light emitting parts 23 are shown in a dot pattern of yet another unique density. The patterns of the first light emitting parts 21, the second light emitting parts 22, and the third light emitting parts 23 vary in the density of dots.

In a top view, the first light emitting parts 21 are positioned in the outer region R2. One or more second light emitting parts 22 are positioned in the center region R1. In a top view, the third light emitting parts 23 are positioned in the middle region R3. The first light emitting parts 21, the second light emitting parts 22, and the third light emitting parts 23 are connected in series with each other via the conductive member 11. The first light emitting parts 21 are not positioned in the center region R1. The second light emitting parts 22 are not positioned in the outer region R2. The first light emitting parts 21 and the second light emitting parts 22 are not positioned in the middle region R3. The third light emitting parts 23 are not positioned in the center region R1 or the outer region R2.

For example, the number of first light emitting parts 21 positioned in the outer region R2 is greater than the number of second light emitting parts 22 positioned in the center region R1. The number of third light emitting parts 23 positioned in the middle region R3 is greater than the number of second light emitting parts 22 positioned in the center region R1 and less than the number of first light emitting parts 21 positioned in the outer region R2.

The first light emitting parts 21, the second light emitting parts 22, and the third light emitting parts 23 are positioned in different regions in the example illustrated in FIG. 3, but have the same configuration. The "same configuration" used herein means exhibiting at least the same wavelength range of emitted light, luminous intensity, and light distribution angle. The first light emitting parts 21, the second light emitting parts 22, and the third light emitting parts 23 emit, for example, ultraviolet light. In this case, the light source module 1 can emit ultraviolet light from the optical member 30. The first light emitting parts 21, the second light emitting parts 22, and the third light emitting parts 23 may emit light of a wavelength range other than that of ultraviolet light.

When the first light emitting parts 21, the second light emitting parts 22, and the third light emitting parts 23 emit ultraviolet light, the peak wavelength of the ultraviolet light is between, for example, 10 nm and 405 nm, inclusive. The difference in the peak wavelength of emitted light is preferably within 5 nm among the first light emitting part 21, the second light emitting part 22, and the third light emitting part 23. The first light emitting parts 21, the second light emitting parts 22, and the third light emitting parts 23 include light emitting elements. The light emitting elements may be, for example, light emitting diodes (LEDs) or laser diodes (LDs). As each of the first light emitting part 21, the second light emitting part 22, and the third light emitting part 23, for example, a light emitting device in which a light emitting element is arranged in a package can be used. The package may be made of a ceramic material and/or a resin material.

The first light emitting part 21, the second light emitting part 22, and the third light emitting part 23 may emit lights of different wavelengths. For example, the first light emitting parts 21, the second light emitting parts 22, and the third light emitting parts 23 may include two or more light emitting parts that emit light having a peak wavelength between 260 nm and 280 nm, inclusive, light emitting parts that emit light having a peak wavelength greater than 280 nm and less than 315 nm, and light emitting parts that emit light having a peak wavelength between 315 nm and 405 nm, inclusive. In one example in which these three types of light emitting parts are used, the first light emitting parts 21 emits light having a peak wavelength between 260 nm and 280 nm, inclusive, the second light emitting parts 22 emits light having a peak wavelength between 315 nm and 405 nm, inclusive, and the third light emitting parts 23 emits light having a peak wavelength greater than 280 nm and less than 315 nm. Also, the peak wavelength of emitted light may be interchanged between the first light emitting parts 21 and the second light emitting parts 22, between the first light emitting parts 21 and the third light emitting parts 23, or between the second light emitting parts 22 and the third light emitting parts 23.

In the examples shown in FIG. 1A to FIG. 3, fourteen first light emitting parts 21 are positioned in the outer region R2. Five second light emitting parts 22 are positioned in the center region R1. Nine third light emitting parts 23 are positioned in the middle region R3. The number of light emitting parts positioned in each region is not limited to the examples shown in FIG. 1A to FIG. 3.

The optical member 30 has a square outer shape in a top view. The optical member 30 may have a rectangular, circular, or elliptical outer shape in a top view. The terms "square" and "rectangle" used herein may encompass ones with chamfered or rounded corners in addition to ones with right-angled corners.

The optical member 30 includes light control parts 35. The optical member 30 has an upper surface and a lower surface, and the light control parts 35 are positioned on the upper surface of the optical member 30. The light control parts 35 are protruding parts that protrude toward a side opposite to the light sources 20. In a top view, the thickness of each protruding part is greatest at its center part and is gradually reduced away from the center part. The thickness of each protruding part refers to the distance from the upper surface of the optical member 30 to the surface of the protruding part in the direction normal to the upper surface of the optical member 30.

The light control parts 35 (protruding parts) can function as convex lenses. In the examples shown in FIG. 1A to FIG. 3, each of the protruding parts has a circular shape in a top view. The optical member 30 can be made of, for example, glass or resin. The glass may be, for example, quartz glass, borosilicate glass, etc. The resin may be, for example, silicone-based resin.

The light control parts 35 includes a plurality of first light control parts 31. In the example shown in FIG. 1A to FIG. 3, the light control parts 35 further include one or more second light control parts 32 and a plurality of third light control parts 33. The first light control parts 31 are positioned in an outer region in the optical member 30 in a top view. The second light control parts 32 are positioned in a center region in the optical member 30 in a top view. The third light control parts 33 are positioned in a middle region, which is located between the center region and the outer region in the optical member 30 in a top view.

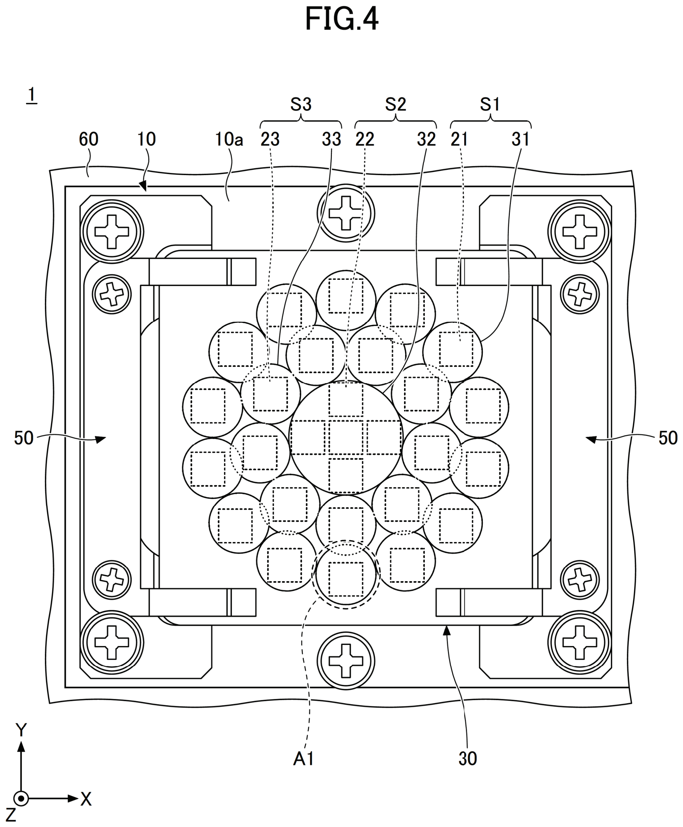

For example, assuming that a single light control part of the light control parts 35 has a circular outer edge in a top view, circles that correspond to such outer edges of adjacent light control parts may or may not have portions overlapping with each other when seen from above. Although the circles corresponding to outer edges of the first light control parts 31 that are adjacent to each other in a top view do not have overlapping portions in the example of FIG. 2, these circles may have overlapping portions. Likewise, although the circles corresponding to outer edges of the third light control parts 33 that are adjacent to each other in a top view have overlapping portions in FIG. 2, these circles do not have to overlap with each other. Although circles corresponding to outer edges of some first light control parts 31 overlap with circles corresponding to outer edges of third light control parts 33 adjacent to them in a top view in FIG. 2, these circles do not have to overlap. Although the circle corresponding to an outer edge of the second light control part 32 and the circles corresponding to outer shapes of third light control parts 33 adjacent to it have no overlapping part in a top view in FIG. 2, the circle of the second light control part and the circles of the third light control parts 33 may overlap with each other. In FIG. 2, it is assumed that an outer edge of a single light control part has a circular shape in a top view, and circular arcs that correspond to overlapping portions of such outer edges of light control parts adjacent to each other are illustrated using dashed lines. The same applies to FIG. 4, FIG. 5, FIG. 6, FIG. 9, FIG. 11, FIG. 12, FIG. 13, FIG. 14, FIG. 16, and FIG. 18, which will be described later.

Light emitted from one of the first light emitting parts 21 is incident on a first light control part 31 that overlaps with it in a top view. A light distribution angle of the incident light is narrowed by the protruding part of the first light control part 31, and the light exits the first light control part 31 on a side (the upper surface) opposite to the substrate 10. Light emitted from one or more of the second light emitting parts 22 is incident on the second light control part 32 that overlaps with them in a top view. A light distribution angle of the incident light is narrowed by the protruding part of the second light control part 32, and the light exits the second light control part 32 on a side opposite to the substrate 10. Light emitted from one of the third light emitting parts 23 that coincides with the third light control part 33 in a top view is incident on each of the third light control parts 33. The light distribution angle of the incident light is narrowed by the protruding part of the third light control part 33, and the light exits the third light control part 33 on a side opposite to the substrate 10.

The thickness of the protruding part may be the same or different among the first light control parts 31, the second light control parts 32, and the third light control parts 33. The top-view area of the protruding part may be the same or different among the first light control parts 31, the second light control parts 32, and the third light control parts 33. The curvature of the protruding part may be the same or different among the first light control parts 31, the second light control parts 32, and the third light control parts 33. The light distribution angle can be adjusted by changing the thicknesses of the protruding part, the area of the protruding part in a top view, and/or the curvature of the protruding part.

In the illustrated examples, each first light control part 31 of the optical member 30 coincides with a unique first light emitting part 21 in a top view, and the second light control part 32 of the optical member 30 overlaps with five second light emitting parts 22 in a top view. With this structure, a light source module with an optical member 30 can be realized, in which the first light emitting parts 21 positioned in the outer region R2 are optically controlled individually by respective first light control parts 31, and in which the five second light emitting parts 22 positioned in the center region R1 are optically controlled collectively by a single second light control part 32.

Also, the light source module 1 may include a plurality of third light emitting parts 23 and multiple third light control parts 33 as needed, and each third light control part 33 may be positioned to overlap with a respective one of third light emitting parts 23 in a top view. With this structure, a light source module having an optical member 30, in which the light sources 20 are formed with first light emitting parts 21, second light emitting parts 22, and third light emitting parts 23 arranged as described above, can be provided.

FIG. 4 is a partial top view (2) showing an example of the light source module according to the first embodiment. As shown in FIG. 4, each first light control part 31 overlaps with a respective one of the first light emitting parts 21 in a top view so as to constitute a first stacked body S1 together. The first stacked bodies S1 formed thus are positioned in the outer region R2 as shown in FIG. 3. Likewise, one or more second light control parts 32 overlap with one or more second light emitting parts 22 in a top view to constitute second stacked bodies S2 such that the number of second light control parts 32 and the number of second stacked bodies S2 are the same. In the illustrated examples, one second light control part 32 overlaps with five second light emitting parts 22 in a top view, constituting one second stacked body S2 together. The second stacked body S2 is positioned in the center region R1 in FIG. 3. Also, in a top view, each third light control part 33 overlaps with a respective one of the third light emitting parts 23 so as to constitute a third stacked body S3 together. The third stacked bodies S3 formed thus are positioned in the middle region R3 shown in FIG. 3.

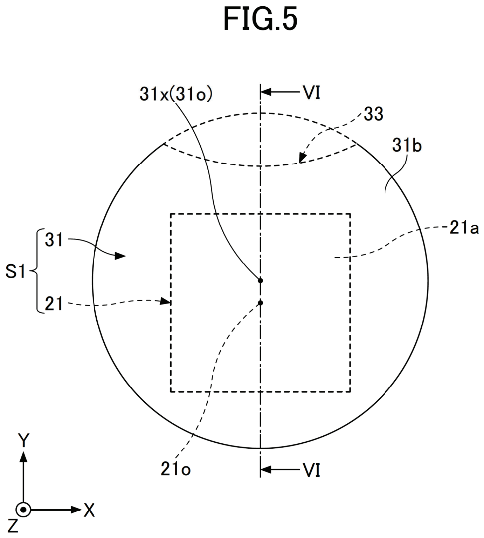

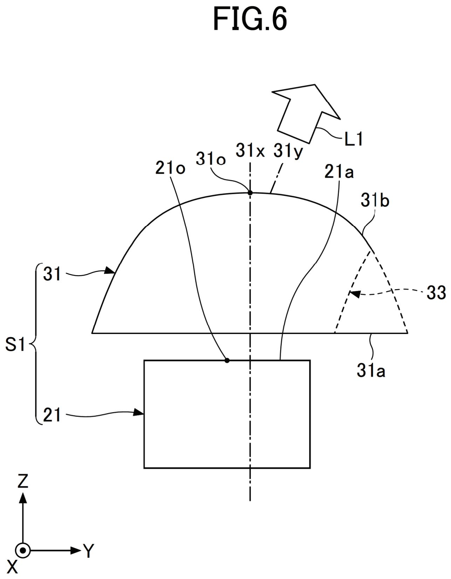

FIG. 5 is a schematic diagram showing an enlarged view of the part labeled "A1" in FIG. 4. FIG. 6 is a schematic diagram showing a cross section of a first stacked body S1, taken along the line VI-VI in FIG. 5. The line VI-VI is parallel to the second direction Y. In FIG. 5 and FIG. 6, "21a" represents a light emitting surface of a first light emitting part 21, and "21o" represents the center of the light emitting surface 21a of the first light emitting part 21 in a top view. Also, "31a" represents a first incident surface of a first light control part 31, "31b" represents a first emission surface of the first light control part 31, and "31o" represents the center of the first emission surface 31b in a top view. Also, "31x" represents a first axis, which passes through the center 31o of the first emission surface 31b and is perpendicular to the first incident surface 31a. "31y" represents the optical axis of the first light control part 31. The optical axis 31y serves as an optical path of light emitted from the center 21o of the light emitting surface 21a of the first light emitting part 21. FIG. 6 is a cross section that passes through the center 21o of the light emitting surface 21a of the first light emitting part 21, and that is along the first axis 31x. The center of a light emitting surface in a top view herein means the geometric center of the light emitting surface in a top view. Similarly, the center of an emission surface in a top view herein means the geometric center of the emission surface in a top view.

As shown in FIG. 5 and FIG. 6, in each first stacked body S1, the first light control part 31 has a first incident surface 31a, which is planar in shape and faces the first light emitting part 21, and a first emission surface 31b, which is curved to be convex in a direction away from the first incident surface 31a. In any cross section along the first axis 31x, both sides of the first emission surface 31b with respect to the first axis 31x have the same curvature. Furthermore, it is preferable that, in all of the cross sections along the first axis 31x, both sides of the first emission surface 31b with respect to the first axis 31x, have the same curvature. In other words, the first light control part 31, in a state of a single first light control part 31 not overlapping a neighboring light control part, is preferably rotationally symmetrical with respect to the first axis 31x. As shown in FIG. 2 and other figures, when circles corresponding to outer edges of adjacent light control parts overlap with each other when viewed from above, such a shape is included in a case in which both sides of the first emission surface 31b with respect to the first axis 31x have the same curvature. In the present specification, the term "the same curvature" herein encompasses a case in which the two curvatures are the same and a case in which the larger one of two curvatures is 102% or less of the smaller one of them. The term "different curvatures" herein may refer to, for example, a case in which the larger one of two curvatures is greater than 102% of the smaller one of them.

In at least one of the first stacked bodies S1, the optical axis 31y of the first control part 31 is non-parallel to the first axis 31x. For example, in at least one of the first stacked bodies S1, when viewed from above, the first axis 31x is separate from the center 21o of the light emitting surface 21a of the first light emitting part 21, and thus the light emitted from the light emitting surface 21a can be refracted at the first emission surface 31b. With this structure, the optical axis 31y can be made non-parallel to the first optical axis 31x. Accordingly, the light source module 1 can easily irradiate light onto a specific region. In the present specification, "parallel" refers to, for example, a case in which the offset between two axes in question is less than 2 degrees. The term "non-parallel" refers to, for example, a case in which the offset between two axes in question is 2 degrees or greater.

For example, in at least one of the first stacked bodies S1, the first axis 31x may or may not be located closer to the center region R1 than is the center 21o of the light emitting surface 21a of the first light emitting part 21 in a top view. With the first axis 31x located closer to the center region R1 than is the center 21o of the light emitting surface 21a of the first light emitting part 21 in a top view in at least one of the first stacked bodies S1, the light source module 1 can easily emit light from the first stacked body S1 toward the center of the center region R1.

In all of the first stacked bodies S1, the optical axis 31y of the constituent first light control part 31 may or may not be non-parallel to the first axis 31x. For example, with the first axis 31x located closer to the center region R1 than is the center 21o of the light emitting surface 21a of the first light emitting part 21 in a top view in all of the first stacked bodies S1, the optical axis 31y can be non-parallel to the first axis 31x. With this structure, the light source module 1 can easily emit light from all of the first stacked bodies S1 toward the center of the center region R1.

The positional relationship between, for example, a first light emitting part 21 and its corresponding first light control part 31 can be adjusted within the range in which the center 21o of the light emitting surface 21a and the first emission surface 31b of the first light control part 31 overlap with each other in a top view.

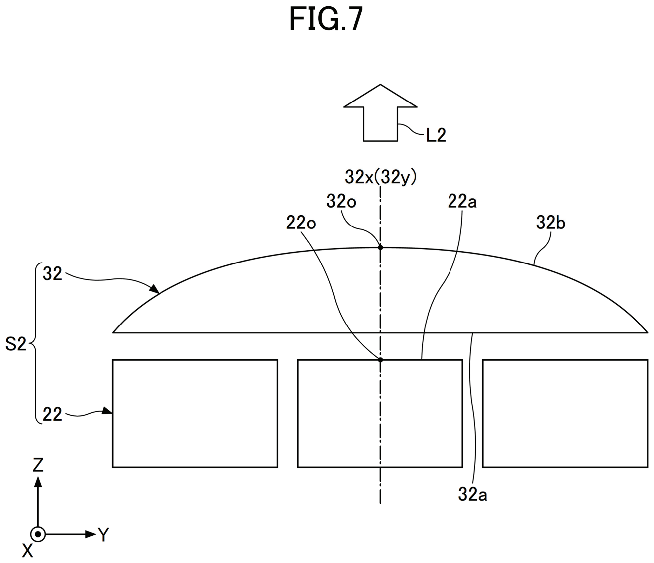

FIG. 7 is a cross-sectional view (1) showing an example of a second stacked body S2. FIG. 7 shows a cross section that passes through the center of a second light control part 32 in a top view in FIG. 2, and that is parallel to the Y-Z plane. Referring to FIG. 7, "22a" represents a light emitting surface of a second light emitting part 22, and "22o" represents the center of the light emitting surface 22a of the second light emitting part 22 viewed from above. Also, "32a" represents a second incident surface of the second light control part 32, "32b" represents a second emission surface of the second light control part 32, and "32o" represents the center of the second emission surface 32b when viewed from above. Also, "32x" represents a second axis, which passes through the center 32o of the second emission surface 32b and is perpendicular to the second incident surface 32a. "32y" represents the optical axis of the second light control part 32. The optical axis 32y serves as a path of the light emitted from the center 22o of the light emitting surface 22a of the second light emitting part 22.

As shown in FIG. 7, in each second stacked body S2, the second light control part 32 has a second incident surface 32a, which is planar in shape and faces the second light emitting part 22, and a second emission surface 32b, which is curved to be convex in a direction away from the second incident surface 32a. In this case, in any cross section along the second axis 32x that passes through the center 32o of the second emission surface 32b and that is perpendicular to the second incident surface 32a in a top view, the curvature of the second emission surface 32b can be the same on both sides of the second emission surface 32b with respect to the second axis 32x. Furthermore, in all of the cross sections along the second axis 32x that passes through the center 32o of the second emission surface 32b and that is perpendicular to the second incident surface 32a in a top view, the curvature of the second emission surface 32b can be made the same on both sides of the second emission surface 32b with respect to the second axis 32x. In other words, the second light control part 32 in a state of a single second light control part 32 not overlapping an adjacent light control part is preferably rotationally symmetrical with respect to the second axis 32x. In the second stacked body S2 shown in FIG. 7, the optical axis of the second light control part 32 is parallel to the third direction Z.

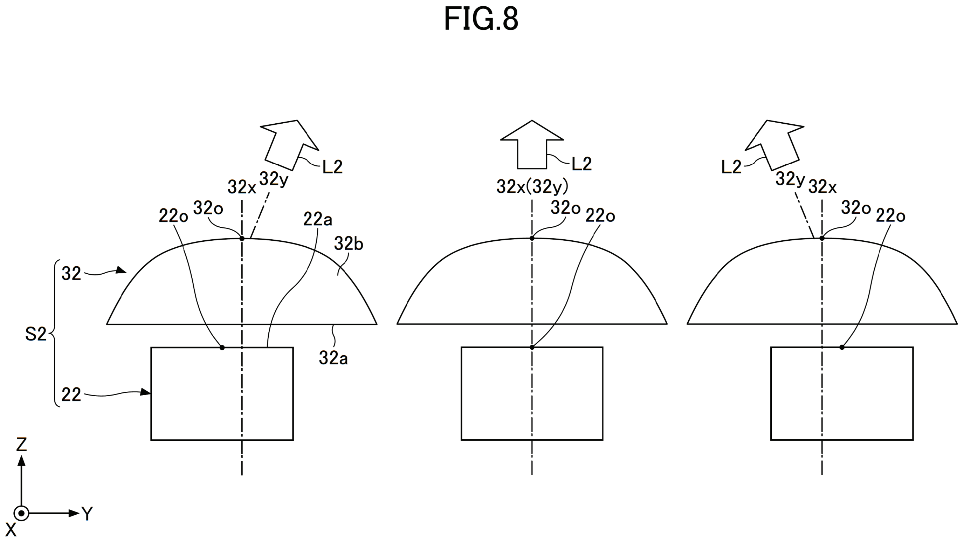

FIG. 8 is a cross-sectional view (2) showing examples of second stacked bodies. Referring to FIG. 8, when a light source module has multiple second stacked bodies S2, the optical axis of the second light control part 32 may be non-parallel to the second axis 32x in all of the second stacked bodies S2, but it is preferable that the optical axis of the second light control part 32 is non-parallel to the second axis 32x in at least one of the second stacked bodies S2. In both cases of FIG. 7 and FIG. 8, it is possible to emit light from each second stacked body S2 toward the center of the center region R1 with ease.

In the light source module 1, all the third stacked bodies S3 emit light in directions parallel to the third direction Z.

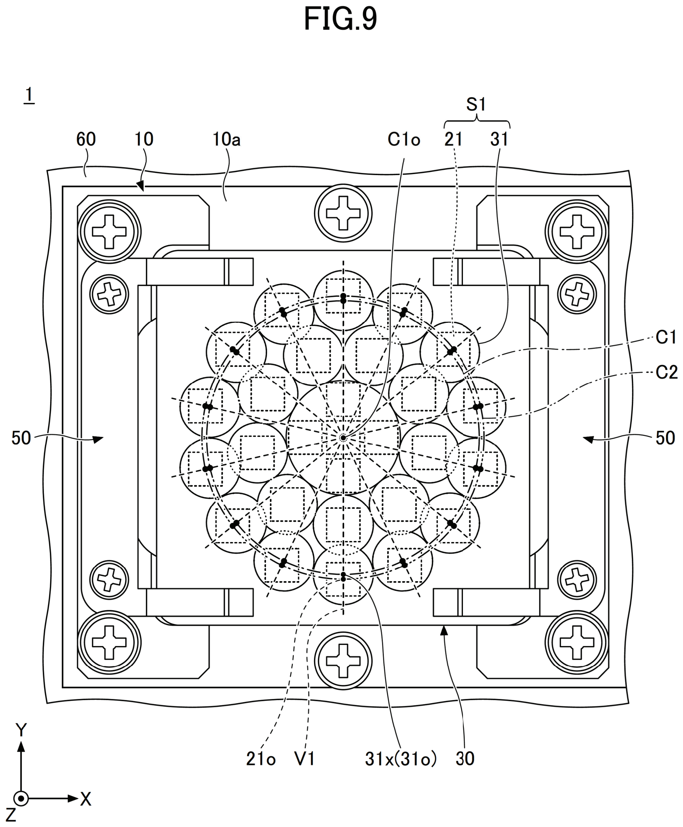

FIG. 9 is a partial top view (3) showing the light source module of the first embodiment. FIG. 9 shows a specific example in which, in each first stacked body S1, the first axis 31x and the center 21o are separate from each other. In the example illustrated in FIG. 9, in all of the first stacked bodies S1, the optical axis 31y of the first light control part 31 is non-parallel to the first axis 31x, and, in a top view, the first axis 31x is located closer to the center region R1 than is the center 21o of the light emitting surface 21a of the first light emitting part 21. In FIG. 9, in all of the first stacked bodies S1, the first axis 31x is positioned on the circumference of a first virtual circle C1 in a top view, and the center 21o of the light emitting surface 21a of the first light emitting part 21 and the first axis 31x are, in a top view, on a first virtual line V1 that passes through the center C1o of the first virtual circle C1. Referring to FIG. 9, in each one of the first stacked bodies S1, in a cross section that passes through the first axis 31x and the first virtual line V1, both sides of the first emission surface 31b with respect to the first axis 31x, have the same curvature.

In FIG. 9, in all of the first stacked bodies S1, in a top view, the distance from the center C1o to the center 21o is longer than the distance from the center C1o to the first axis 31x. Furthermore, in FIG. 9, the distance from the center C1o to the center 21o is equal in all of the first stacked bodies S1 in a top view. In other words, the respective centers 21o are all on the circumference of a virtual circle C2. The center of the virtual circle C2 and the center C1o of the first virtual circle C1 are the same. The radius of the virtual circle C2 is larger than the radius of the first virtual circle C1. Thus, when seen from above, all the first axes 31x are positioned on the circumference of the first virtual circle C1 and all the centers 21o are positioned on the circumference of the virtual circle C2, so that the lights emitted from individual first stacked bodies S1 can be concentrated in a narrower area.

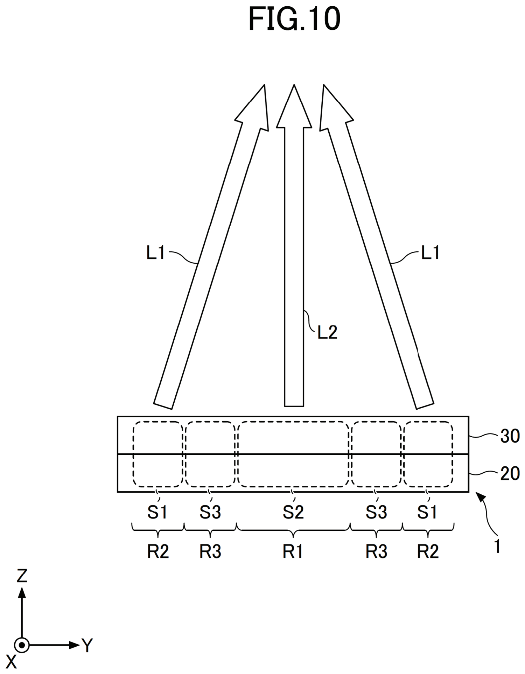

In the example of FIG. 9, the light source module 1 can emit light from the first stacked bodies S1 towards the center of the center region R1 with ease, as schematically illustrated in FIG. 10. Also, the second stacked body S2 can easily emit light toward the center of the center region R1. This is effective when, for example, an object to be irradiated with light exists in the direction through the center of the center region R1.

In each first stacked body S1, in a top view, the first axis 31x may be located farther from the center region R1 than is the center 21o of the light emitting surface 21a of the first light emitting part 21. For example, in the example of FIG. 9, the radius of the virtual circle C2 can be smaller than the radius of the first virtual circle C1. With this structure, the first stacked bodies S1 can easily emit light in a direction diverging from the center region R1. This is effective when, for example, there is an object to be irradiated with light in a direction diverging from the center region R1.

### <First Modified Example of First Embodiment>

In a first modified example of the first embodiment, an example light source module will be shown, in which the positional relationship between the third light emitting part 23 and the third light control part 33 in the third stacked bodies S3 is different from that of the first embodiment. According to the first modified example of the first embodiment, the positional relationship between the first light emitting part 21 and the first light control part 31 in the first stacked bodies S1 and the positional relationship between the second light emitting parts 22 and the second light control part 32 in the second stacked body S2 are the same as in the first embodiment.

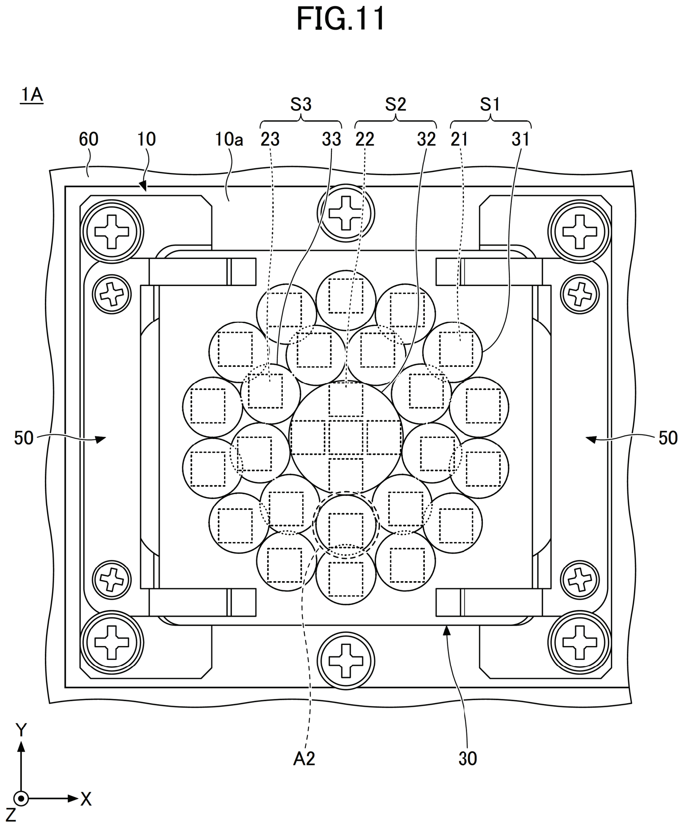

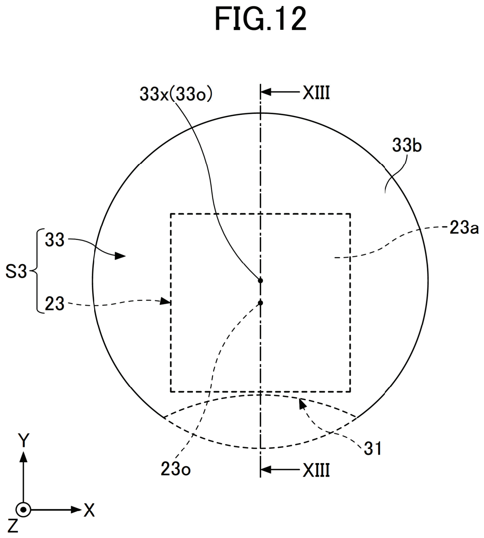

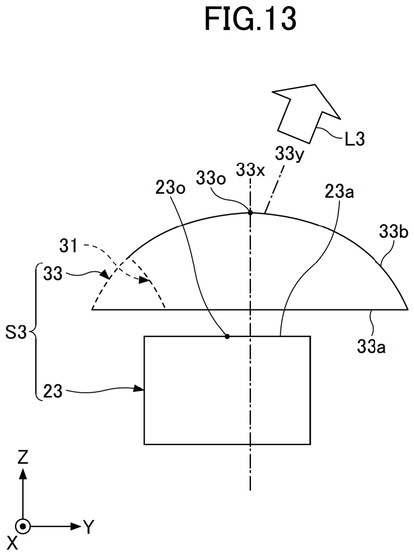

FIG. 11 is a partial top view (1) showing an example of a light source module 1A according to the first modified example of the first embodiment. FIG. 12 is a schematic diagram showing an enlarged view of the part labeled "A2" in FIG. 11. FIG. 13 is a schematic diagram showing a cross section of a third stacked body S3, taken along the line XIII-XIII in FIG. 12. The line XIII-XIII is parallel to the second direction Y.

In FIG. 12 and FIG. 13, "23a" represents a light emitting surface of a third light emitting part 23, and "23o" represents the center of the light emitting surface 23a of the third light emitting part 23 in a top view. Also, "33a" represents a third incident surface of a third light control part 33, "33b" represents a third emission surface of the third light control part 33, and "33o" represents the center of the third emission surface 33b in a top view. Also, "33x" represents a third axis, which passes through the center 33o of the third emission surface 33b and perpendicular to the third incident surface 33a. "33y" represents the optical axis of the third light control parts 33. The optical axis 33y serves as a path of the light emitted from the center 23o of the light emitting surface 23a of the third light emitting part 23. FIG. 13 is a cross section that passes through the center 23o of the light emitting surface 23a of the third light emitting part 23 and that is along the third axis 33x.

As shown in FIG. 12 and FIG. 13, in each third stacked body S3 of the light source module 1A, the third light control part 33 has: a third incident surface 33a that is planar in shape and that faces the third light emitting part 23; and a third emission surface 33b that is curved to be convex in a direction away from the third incident surface 33a. In any cross section along the third axis 33x, both sides of the third emission surface 33b with respect to the third axis 33x have the same curvature. Furthermore, it is preferable that, in all of the cross sections along the third axis 33x, both sides of the third emission surface 33b with respect to the third axis 33x have the same curvature. In other words, the third light control part 33 in a state of a single third light control part 33 not overlapping a neighboring light control part is preferably rotationally symmetrical with respect to the third axis 33x.

In at least one of the third stacked bodies S3, the optical axis 33y of the third light control part 33 is non-parallel to the third axis 33x. For example, in at least one of the third stacked bodies S3, with the third axis 33x separate from the center 23o of the light emitting surface 23a of the third light emitting part 23 in a top view, the light emitted from the light emitting surface 23a can be refracted at the third emission surface 33b. With this structure, the optical axis 31y can be non-parallel to the first optical axis 31x.

In at least one of the third stacked bodies S3, the direction in which the center 23o of the light emitting surface 23a is separated from the third axis 33x preferably coincides with the direction in which the center 21o of the light emitting surface 21a is separated from the first axis 31x in the first stacked bodies S1. In other words, in at least one of the third stacked bodies S3, it is preferable that, in a top view, the first axis 31x is located closer to the center region R1 than the center 21o of the light emitting surface 21a of the first light emitting part 21 is to the center region R1. This structure can facilitate emission of light from at least one third stacked body S3 towards the center of the center region R1.

In all of the third stacked bodies S3, the optical axis 33y of the third light control part 33 may or may not be non-parallel to the third axis 33x. For example, in all of the third stacked bodies S3, the third axis 33x is located closer to the center region R1 than is the center 23o of the light emitting surface 23a of the third light emitting part 23 in a top view, so that the optical axis 33y can be made non-parallel to the first axis 33x. With this structure, in the light source module 1A, light can be easily emitted from all of the third stacked bodies S3 toward the center of the center region R1.

For example, the positional relationship between a third light emitting part 23 and its corresponding third light control part 33 can be adjusted within the range in which the center 23o of the light emitting surface 23a and the third emission surface 33b of the third light control part 33 coincide with each other in a top view.

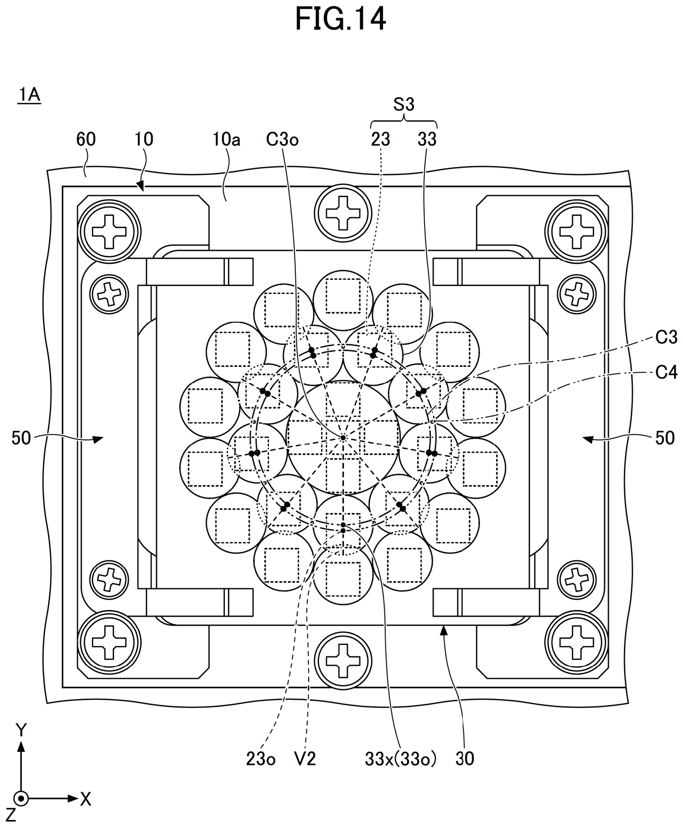

FIG. 14 is a partial top view (2) showing the light source module according to the first modified example of the first embodiment. FIG. 14 shows a specific example in which the third axis 33x and the center 23o are separate from each other. In the example illustrated in FIG. 14, in all of the third stacked bodies S3, the optical axis 33y of the third light control part 33 is non-parallel to the third axis 33x, and, when seen from above, the third axis 33x is located closer to the center region R1 than the center 23o of the light emitting surface 23a of the third light emitting part 23 is to the center region R1. In FIG. 14, in all of the third stacked bodies S3, the third axis 33x is positioned on the circumference of a second virtual circle C3 in a top view, and the center 23o of the light emitting surface 23a of the third light emitting part 23 and the third axis 33x are, in a top view, on a second virtual line V2 that passes through the center C3o of the second virtual circle C3. It is preferable that the center C3o of the second virtual circle C3 coincides with the center C1o of the first virtual circle C1 shown in FIG. 9. Referring to FIG. 14, in each of the third stacked bodies S3, in a cross section that passes through the third axis 33x and the second virtual line V2, both sides of the third emission surface 33b with respect to the third axis 33x, have the same curvature.

In FIG. 14, in all of the third stacked bodies S3, in a top view, the distance from the center C3o to the center 23o is longer than the distance from the center C3o to the third axis 33x. Furthermore, in FIG. 14, the distance from the center C3o to the center 23o is equal in all of the third stacked bodies S3 in a top view. In other words, all the centers 23o are positioned on the circumference of a virtual circle C4. The center of the virtual circle C4 and the center C3o of the second virtual circle C3 are the same. The radius of the virtual circle C4 is larger than the radius of the second virtual circle C3. Thus, when seen from above, all the third axes 33x are positioned on the circumference of the second virtual circle C3 and all the centers 23o are positioned on the circumference of the virtual circle C4, so that the lights emitted from individual third stacked bodies S3 can be concentrated in a narrower area.

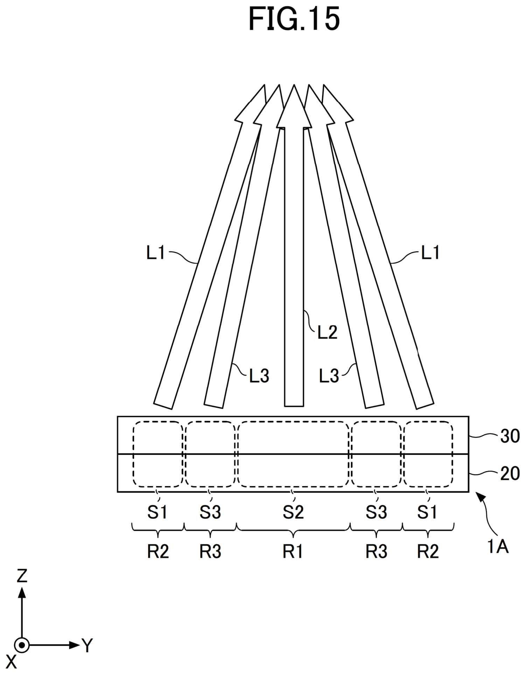

As schematically illustrated in FIG. 15, the configuration shown in FIG. 14 can facilitate emission of light from the first stacked bodies S1 toward the center of the center region R1, emission of light from the second stacked body S2 toward the center of the center region R1, and emission of light from the third stacked bodies S2 toward the center of the center region R1. In other words, the light source module 1A can concentrate a greater amount of light towards the center of the center region R1 than the light source module 1.

### <Second Modified Example of First Embodiment>

In a second modified example of the first embodiment, an example light source module will be shown, in which the positional relationship between the first light emitting part 21 and the first light control part 31 in the first stacked bodies S1 is different from that of the first embodiment. According to the first modified example of the first embodiment, the positional relationship between the second light emitting parts 22 and the second light control part 32 in the second stacked body S2 and the positional relationship between the third light emitting part 23 and the third light control part 33 in the third stacked bodies S3 are the same as those in the first embodiment.

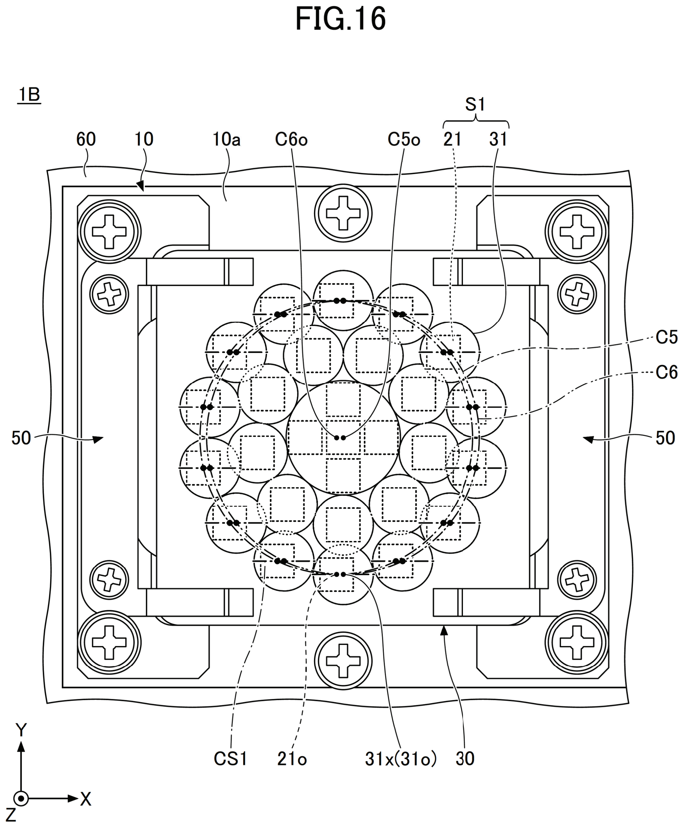

FIG. 16 is a partial top view showing an example of a light source module 1B according to the second modified example of the first embodiment. As shown in FIG. 16, in all of the first stacked bodies S1 of the light source module 1B, the first axis 31x is separate from the center 21o of the light emitting surface 21a of the first light emitting part 21 in a top view. Furthermore, the first cross sections CS1 of all first stacked bodies S1, passing through the center 21o of the light emitting surface 21a of the first light emitting part 21 and taken along the first axis 31x, are parallel to each other in a top view. The position of the center 21o of the light emitting surface 21a of the first light emitting part 21 relative to the first axis 31x is in the same direction across the first cross sections CS1. In the example illustrated, all the first cross sections CS1 are parallel to the first direction X. In all of these first cross sections CS1, the center 21o of the light emitting surface 21a of the first light emitting part 21 is located in the -X direction with respect to the first axis 31x.

The respective extending lines of the first cross sections CS1 of the first stacked bodies S1 of the light source module 1B may or may not coincide with each other in a top view.

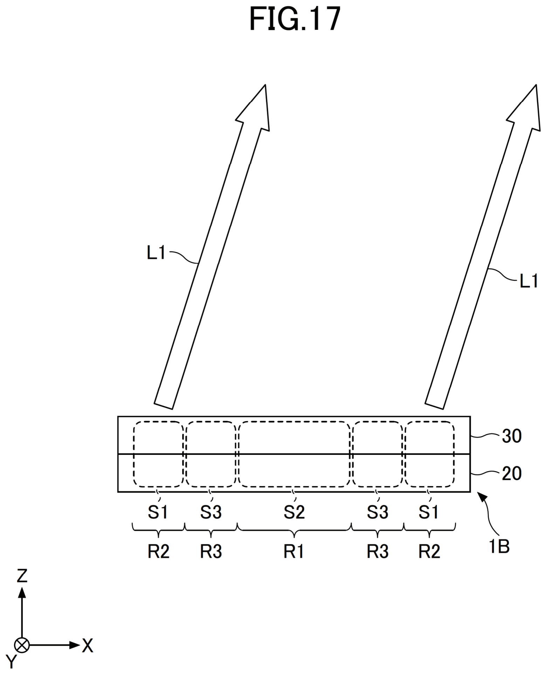

In the example illustrated in FIG. 16, every first stacked body S1 can easily emit light diagonally upward in the +X direction, as shown in FIG. 17. The light emitted from each first stacked body S1 has its optical axis on its corresponding first cross section CS1, and travels from the -X side to the +X side in a top view. In the light source module 1B, all the first cross sections CS1 are parallel to a predetermined direction in a top view, so that light can be emitted from each first stacked body S1 in a predetermined direction that is parallel to the first cross section CS1 in a top view.

### <Third Modified Example of First Embodiment>

In a third modified example of the first embodiment, an example light source module will be shown, in which the positional relationship between the third light emitting part 23 and the third light control part 33 in the third stacked bodies S3 is different from that of the second modified example of the first embodiment. In the third modified example of the first embodiment, the positional relationship between the first light emitting part 21 and the first light control part 31 in the first stacked bodies S1 and the positional relationship between the second light emitting parts 22 and the second light control part 32 in the second stacked body S2 are the same as those in the second modified example of the first embodiment.

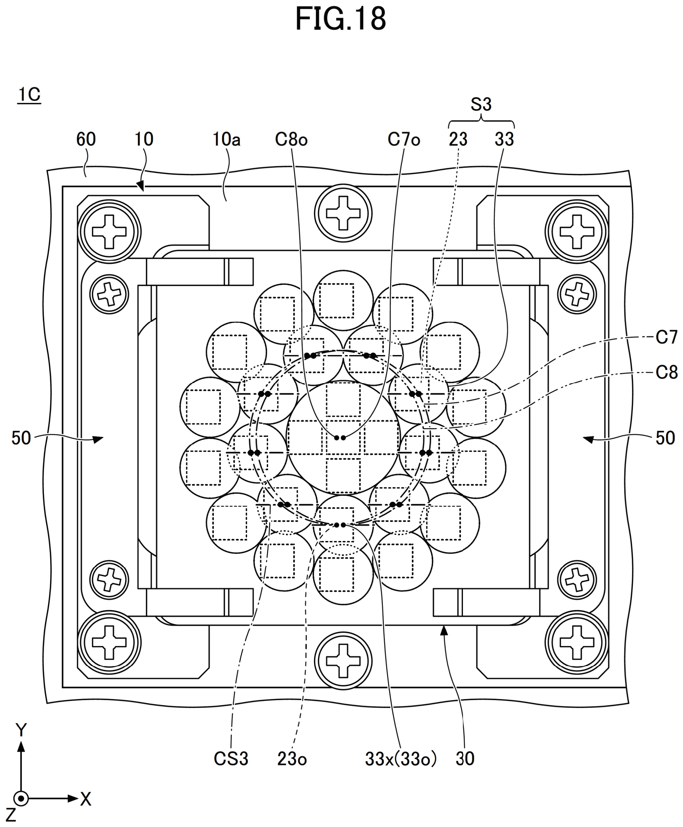

FIG. 18 is a partial top view showing an example of a light source module 1C according to the third modified example of the first embodiment. As shown in FIG. 18, in all of the third stacked bodies S3 of the light source module 1C, the third axis 33x is separate from the center 23o of the light emitting surface 23a of the third light emitting part 23 in a top view. Furthermore, in all of the third stacked bodies S3, in a top view, the third cross section CS3 passing through the center 23o of the light emitting surface 23a of the third light emitting part 23 and the third axis 33x is parallel to the first cross section CS1 shown in FIG. 16. In every third cross section CS3, the center 23o of the light emitting surface 23a of the third light emitting part 23 is located in the same direction relative to the third axis 33x. In the example illustrated, all the third cross sections CS3 are parallel to the first direction X. In each of these third cross sections CS3, the center 23o of the light emitting surface 23a of the third light emitting part 23 is located in the -X direction with respect to the first axis 31x.

The respective extending lines of the third cross section CS3 of the third stacked bodies S3 of the light source module 1C may or may not overlap with each other in a top view.

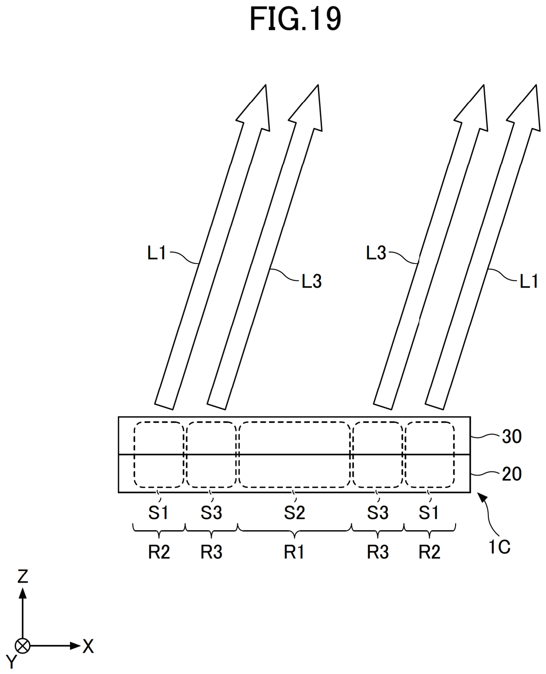

In the example illustrated in FIG. 18, each first stacked body S1 and each third stacked body S3 can easily emit light diagonally upward in the +X direction, as shown in FIG. 19. The light emitted from every first stacked body S1 or every third stacked body S3 has its optical axis on its corresponding first cross section CS1 or third cross section CS3, and travels from the -X direction end to the +X direction end in a top view. In the light source module 1C, all the first cross sections CS1 and all the third cross section CS3 are made parallel to a predetermined direction in a top view, so that light can be emitted from every first stacked body S1 and from every third stacked body S3, in a predetermined direction that is parallel to the first cross section CS1 or the third cross section CS3 in a top view. In other words, the light source module 1C can emit a greater amount of light in a predetermined direction that is parallel to the first cross section CS1, in a top view, than the light source module 1B.

The optical members 30 can be shaped the same in the light source modules 1, 1A, 1B, and 1C. Furthermore, by adjusting the location of the light sources 20 and changing the positional relationship between the light control part 35 of the optical member 30 and the light sources 20, the direction of light emission from the light source module can be changed. The optical member 30 can be produced, for example, by molding using a mold. Even if multiple types of light source modules are to be produced, one mold suffices to produce the optical member 30 for each light source module, so that an increase in the cost of the light source module can be inhibited.

### <Fourth Modified Example of First Embodiment>

In a fourth modified example of the first embodiment, an example to use separate light control parts will be described. In the description given thus far, examples have been illustrated in which an optical member having a plurality of light control parts is used in the light source module. However, it is also possible to use, in a light source module, a plurality of optical members each having one light control part.

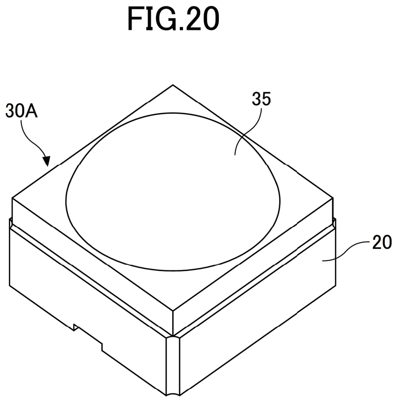

FIG. 20 is a perspective view showing an optical member for use in the light source module according to the fourth modified example of the first embodiment. As shown in FIG. 20, an optical member 30A has one light control part 35 that is positioned above one light-emitting part of the light sources 20. The positional relationship between the light sources 20 and the light control part 35 can be adjusted as appropriate.

The light sources 20 may be, for example, a light emitting device in which one light emitting element is provided in a package. In this case, the first stacked bodies S1 and third stacked bodies S3 can be formed by using optical members 30A. The light sources 20 may also be a light emitting device in which one or more light emitting elements are provided in a package. In this case, the second stacked body S2 can be formed by using an optical member 30A. By positioning these optical members 30A, for example, as shown in FIG. 2, a light source module can be formed.

### <Second Embodiment>

In a second embodiment of the present disclosure, an example of an ultraviolet light fluid processing device having a light source module according to the first embodiment will be shown.

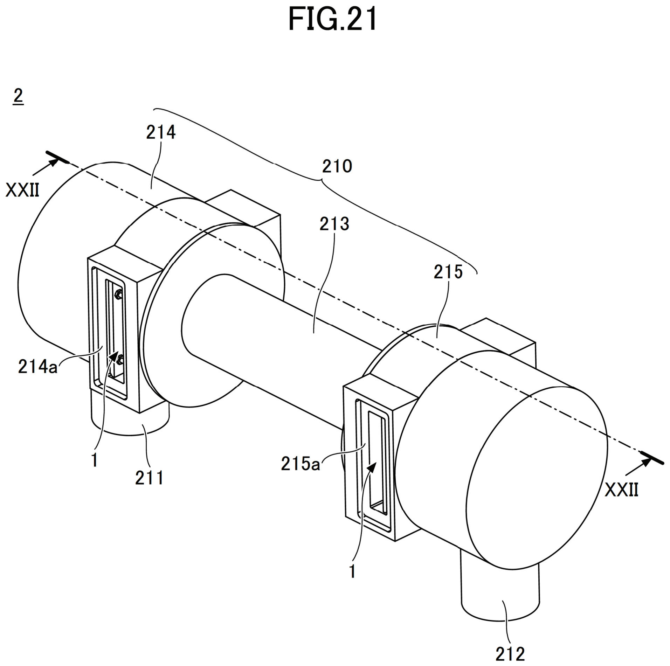

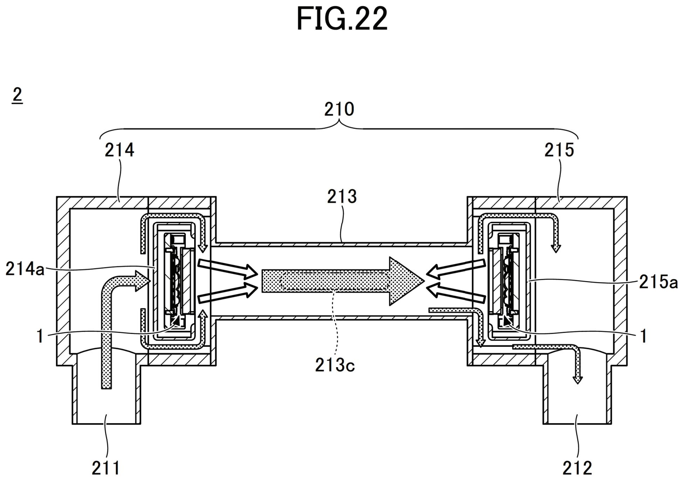

FIG. 21 is a perspective view showing an example of the ultraviolet light fluid processing device according to the second embodiment. FIG. 22 is a cross-sectional view taken along the line XXII-XXII in FIG. 21. Note that, in FIG. 22, the dotted arrows are schematic representations of the flow of fluid. Also, the blank arrows (arrows without dots) are schematic representations of the directions in which the light source module emits ultraviolet light.

As shown in FIG. 21 and FIG. 22, the ultraviolet light fluid processing device 2 has a flow pipe 210, in which fluid flows, and two light source modules 1 that can irradiate the ultraviolet light exiting the optical member 30 (that is, emit ultraviolet light to) onto the inside of the flow pipe 210. The ultraviolet light fluid processing device 2 may include one or three or more light source modules 1.

The flow pipe 210 includes: a flow-in part 211 and a flow-out part 212 where fluid enters and exits the flow pipe 210, respectively; a flow part 213 in which the fluid flows; a first connecting part 214 connecting the flow-in part 211 and the flow part 213; and a second connecting part 215 connecting the flow part 213 and the flow-out part 212. Fluid enters the flow pipe 210 from the flow-in part 211, travels across the flow part 213, and exits from the flow-out part 212. The direction in which the fluid flows may be reversed.

The first connecting part 214 is provided with a light source positioning part 214a for inserting one of the light source modules 1. The light source positioning part 214a is positioned such that the direction in which the one of the light source modules 1 is inserted in the light source positioning part 214a is substantially perpendicular to the direction in which the fluid enters the flow pipe 210 from the flow-in part 211, and the optical members 30 of the inserted light source module 1 face the flow part 213. The one light source module 1 is thus inserted in the light source positioning part 214a and fixed in the light source positioning part 214a by the spring members 70 (see FIG. 1A and FIG. 1B).

The second connecting part 215 is provided with a light source positioning part 215a for inserting the other of the light source modules 1. The light source positioning part 215a is positioned such that the direction in which the other light source module 1 is inserted in the light source positioning part 215a is substantially perpendicular to the direction in which the fluid exits the flow pipe 210 from the flow-out part 212, and the optical members 30 of the inserted light source module 1 face the flow part 213. The other light source module 1 is inserted in the light source positioning part 215a and fixed in the light source positioning part 215a by the spring members 70 (see FIG. 1A and FIG. 1B).

The lower surface of the holding member 60 constituting one light source module 1 can be positioned in surface contact with an inner wall of the light source positioning part 214a (see FIG. 1A and FIG. 1B). Likewise, the lower surface of the holding member 60 constituting the other light source module 1 can be positioned in surface contact with an inner wall of the light source positioning part 215a (see FIG. 1 A and FIG. 1B). With this structure, the heat produced while the light source modules are in operation can be dissipated to the fluid flowing around the light source positioning parts.

The flow part 213 extends in the direction normal to the upper surface 10a of the substrate 10 of one light source module 1 (see FIG. 1A and FIG. 1B). Also, the flow part 213 extends in the direction normal to the upper surface 10a of the substrate 10 of the other light source module 1 (see FIG. 1A and FIG. 1B). Each light source module 1 can irradiate the ultraviolet light exiting the optical members 30 onto the inside of the flow part 213.

Thus, in the examples shown in FIG. 21 and FIG. 22, the ultraviolet light fluid processing device 2 can emit ultraviolet light, from the two light source modules 1, to the fluid in the flow part 213. With this structure, the ultraviolet light fluid processing device 2 can process the fluid by irradiating the fluid in the flow part 213 with ultraviolet light, thereby, for example, reducing the number of bacteria and viruses in the processed water compared to before the processing. Note that "fluid" used herein may include liquids, gases, etc.

The flow-in part 211 and the flow part 213 are located opposite each other with respect to the light source positioning part 214a. Between the outer walls of the light source positioning part 214a and the inner walls of the first connecting part 214, an upper gap and a lower gap that are opposite each other with respect to the light source positioning part 214a are formed. The fluid enters the first connecting part 214 from the flow-in part 211, travels in the upper and lower gaps formed opposite each other with respect to the light source positioning part 214a, and enters the flow part 213.

Also, the flow-out part 212 and the flow part 213 are located opposite each other with respect to the light source positioning part 215a. Between the outer walls of the light source positioning part 215a and the inner walls of the second connecting part 215, an upper gap and a lower gap that are opposite each other with respect to the light source positioning part 215a are formed. The fluid enters the second connecting part 215 from the flow part 213, travels in the upper and lower gaps formed opposite each other with respect to the light source positioning part 215a, and enters the flow-out part 212.

Looking at the flow rate (i.e., speed) of the fluid in the flow part 213, the flow rate in the center part in the flow part 213 may be faster than the flow rate in the outer parts, or the flow rate in the outer parts in the flow part 213 may be faster than the flow rate in the center part. Referring to the example of FIG. 22, the branch of the fluid that flows in the upper gap formed above the upper outer wall of the light source positioning part 214a and then enters the flow part 213 and the branch of the fluid that flows in the lower gap formed below the lower outer wall of the light source positioning part 214a and then enters the flow part 213 merge in the center part 213c in the flow part 213, so that the flow rate in the center part 213c is faster than the flow rate in the outer parts in the flow part 213. By using the light source modules 1 that can collect light in the center part 213c in the flow part 213 thus, an increased amount of ultraviolet light can be collected in the center part 213c in the flow part 210, thereby improving the sterilization performance of the ultraviolet light fluid processing device 2. The light source modules 1A may be used instead of the light source modules 1. By using light source modules 1A, a further increased amount of ultraviolet light can be collected in the center part 213c in the flow part 213, thereby further improving the sterilization performance of the ultraviolet light fluid processing device 2.

Also, by using the light source module 1, light is collected in the center part 213c in the flow part 213, so that vignetting caused by the light source positioning parts can be reduced.

### <First Modified Example of Second Embodiment>

In a first modified example of the second embodiment, an ultraviolet light fluid processing device with different light source modules from those of the second embodiment described above will be described. In the ultraviolet light fluid processing device of the first modified example of the second embodiment, the direction in which the light source modules are inserted, relative to the direction in which fluid flows in and out of the flow part 210, is different from that in the ultraviolet light fluid processing device of the second embodiment described above.

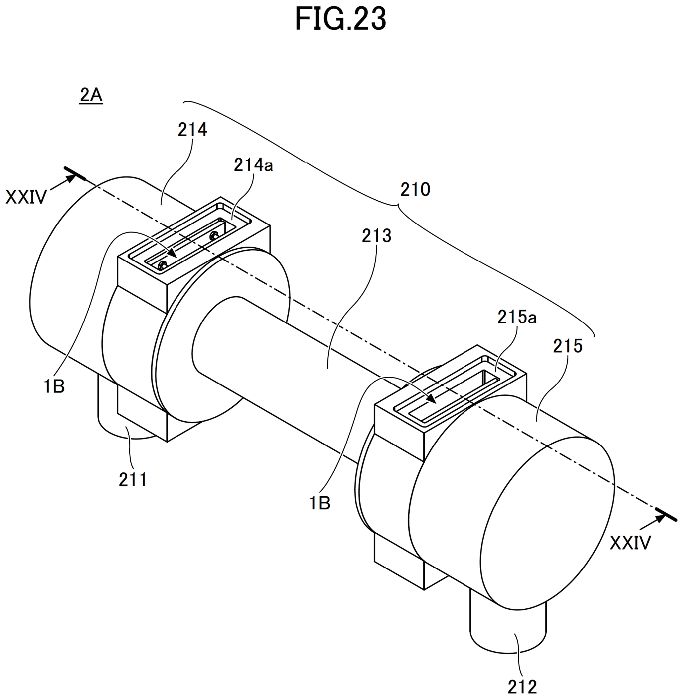

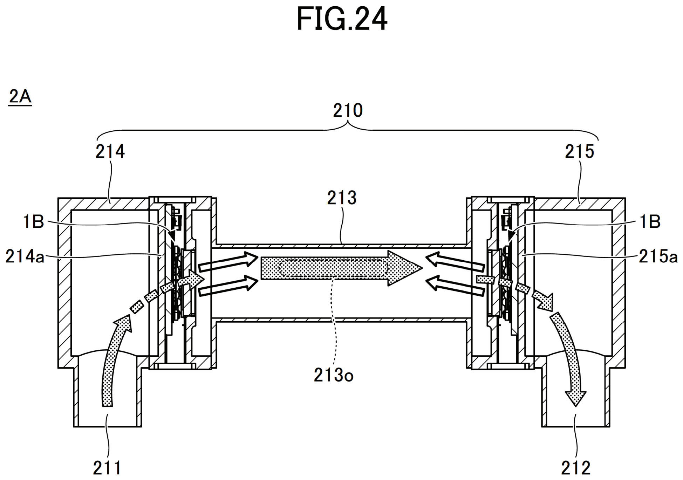

FIG. 23 is a perspective view showing an example of the ultraviolet light fluid processing device according to the first modified example of the second embodiment. FIG. 24 is a cross-sectional view taken along the line XXIV-XXIV in FIG. 23. Note that, in FIG. 24, the dotted arrows are schematic representations of the flow of fluid. Also, the blank arrows (arrows without dots) are schematic representations of the directions in which the light source modules emit ultraviolet light. Referring to FIG. 23 and FIG. 24, the ultraviolet light fluid processing device 2A is different from the ultraviolet light fluid processing device 2 in that the two light source modules 1 of the ultraviolet light fluid processing device 2 are replaced with two light source modules 1B.

Also, unlike the ultraviolet light fluid processing device 2, the light source positioning part 214a of the ultraviolet light fluid processing device 2A is positioned such that the direction in which one light source module 1B is inserted in the light source positioning part 214a is substantially parallel to the direction in which fluid enters the flow pipe 210 from the flow-in part 211, and the optical members 30 of the inserted light source module 1B are oriented to face the flow part 213. Also, the light source positioning part 215a is positioned such that the direction in which the other light source module 1B is inserted in the light source positioning part 215a is substantially parallel to the direction in which the fluid exits the flow pipe 210 from the flow-out part 212, and the optical members 30 of the inserted light source module 1B are oriented to face the flow part 213.

In the example of FIG. 24, the flow-in part 211 and the flow part 213 are located opposite each other with respect to the light source positioning part 214a. Between the outer walls of the light-source positioning part 214a and the inner walls of the first connecting part 214, a front gap and a rear gap that are opposite each other are formed in front of and behind the light source positioning part 214a. The fluid enters the first connecting part 214 from the flow-in part 211, travels in the front and rear gaps formed opposite each other in front of and behind the light source positioning part 214a, and enters the flow part 213.

Also, the flow-out part 212 and the flow part 213 are located opposite each other with respect to the light source positioning part 215a. Between the outer walls of the light source positioning part 215a and the inner walls of the second connecting part 215, a front gap and a rear gap that are opposite each other are formed in front of and behind the light source positioning part 215a. The fluid enters the second connecting part 215 from flow part 213, travels in the front and rear gaps formed opposite each other with respect to the light source positioning part 215a, and enters the flow-out part 212.

The fluid enters the first connecting part 214 from the flow-in part 211, travels in front of and behind the outer walls of the light source positioning part 214a, and enters the flow part 213. Referring to FIG. 24, the arrows' dashed parts represent the branches of fluid that travel in front of and behind the outer walls of the light source positioning part 214a. The branch of the fluid that flows in the front gap formed in front of the outer walls of the light source positioning part 214a and then enters the flow part 213 and the branch of the fluid that flows in the rear gap formed behind the outer walls of the light source positioning part 214a and then enters the flow part 213 merge in the outer-circumferential upper part 213o in the flow part 213, so that the flow rate in the outer-circumferential upper part 213o is faster than the flow rate in the center part and the outer-circumferential lower part in the flow part 213. The above-mentioned merging occur in the outer-circumferential upper part 213o in the flow part 213 because no branch of fluid travels above the upper outer wall of the light source positioning part 214a and then enters the flow part 213 as is the case of the example of FIG. 22. By using the light source modules 1B that can concentrate light in the outer-circumferential upper part 213o in the flow part 213 thus, an increased amount of ultraviolet light can be collected in the outer-circumferential upper part 213 in the flow part 210, thereby allowing for improvement in the sterilization performance of the ultraviolet light fluid processing device 2A. Note that light source modules 1C may be used instead of the light source modules 1B. By using light source modules 1C, a further increased amount of ultraviolet light can be collected in the outer-circumferential upper part 213 in the flow part 210, thereby further improving the sterilization performance of the ultraviolet light fluid processing device 2A.

Preferred embodiments of the present disclosure, as well as their modified examples, have been described in detail above. However, the present disclosure is not limited to the above-described embodiments and modified examples, and a variety of modifications, substitutions, etc. can be made to the embodiments and modified examples without departing from the technical scope defined by the accompanying claims.

For example, the light control parts described herein may be cylindrical lenses or Fresnel lenses.

## Claims

1. A light source module comprising:
a substrate having a center region and an outer region located outward of the center region;
light sources positioned on an upper surface of the substrate and comprising a plurality of first light emitting parts; and
an optical member positioned above the substrate such that the light sources are located between the substrate and the optical member, the optical member comprising light control parts that comprise a plurality of first light control parts,
wherein each of the plurality of first light control parts overlaps with a respective one of the plurality of first light emitting parts in a top view so as to constitute a respective one of a plurality of first stacked bodies, positioned in the outer region,
wherein, in each of the plurality of first stacked bodies, the first light control part comprises:
a first incident surface that is planar in shape and that faces the first light emitting part; and
a first emission surface that is curved to be convex in a direction away from the first incident surface,
wherein, in each of the plurality of first stacked bodies, in any cross section along a first axis that passes through a center of the first emission surface in the top view and that is perpendicular to the first incident surface, both sides of the first emission surface with respect to the first axis have a same curvature, and
wherein, in at least one of the plurality of first stacked bodies, an optical axis of the first light control part is non-parallel to the first axis.

2. The light source module according to claim 1, wherein, in at least one of the plurality of first stacked bodies, the first axis is separate from a center of a light emitting surface of a corresponding one of the plurality of first light emitting parts in the top view.

3. The light source module according to claim 2, wherein, in at least one of the plurality of first stacked bodies, the first axis is located closer to the center region than the center of the light emitting surface of the corresponding first light emitting part is to the center region in the top view.

4. The light source module according to claim 3, wherein, in each of the plurality of first stacked bodies:
the optical axis of the first light control part is non-parallel to the first axis; and
the first axis is located closer to the center region than is the center of the light emitting surface of the corresponding first light emitting part to the center region in the top view.

5. The light source module according to claim 4, wherein, in each one of the plurality of first stacked bodies:
first axes of all of the plurality of first stacked bodies are positioned on a circumference of a first virtual circle in the top view; and
in the top view, the first axis and the center of the light emitting surface of the corresponding first light emitting part are positioned on a first virtual line that passes through a center of the first virtual circle.

6. The light source module according to claim 5, wherein, in each of the plurality of first stacked bodies, both sides of the first emission surface with respect to the first axis have a same curvature in a cross section including the first virtual line.

7. The light source module according to one of claim 1 to claim 6,
wherein the light sources further comprises one or more second light emitting parts,
wherein the light control parts further include one or more second light control parts,
wherein the one or more second light control parts overlap with the one or more second light emitting parts in the top view so as to constitute one or more second stacked bodies, a number of the one or more second stacked bodies being the same as the number of the one or more second light control parts,
wherein the second stacked bodies are positioned in the center region,
wherein, in each of the second stacked bodies, the second light control part comprises:
a second incident surface that is planar in shape and that faces a corresponding second light emitting part of the one or more second light emitting parts; and
a second emission surface that is curved to be convex in a direction away from the second incident surface, and
wherein, in each of the second stacked bodies, in any cross section along a second axis that passes through a center of the second emission surface in the top view and that is perpendicular to the second incident surface, both sides of the second emission surface with respect to the second axis have a same curvature.

8. The light source module according to claim 7,
wherein the light sources further include a plurality of third light emitting parts,
wherein the light control parts further include a plurality of third light control parts,
wherein each of the plurality of third light control parts overlaps with a respective one of the plurality of third light emitting parts in the top view so as to constitute a respective one of a plurality of third stacked bodies,
wherein the substrate has a middle region between the center region and the outer region,
wherein the plurality of third stacked bodies are positioned in the middle region,
wherein, in each of the plurality of third stacked bodies, the third light control part comprises:
a third incident surface that is planar in shape and that faces a corresponding third light emitting part of the plurality of third light emitting parts; and
a third emission surface that is curved to be convex in a direction away from the third incident surface,
wherein, in each of the plurality of third stacked bodies, in any cross section taken along a third axis that passes through a center of the third emission surface in the top view and that is perpendicular to the third incident surface, both sides of the third emission surface with respect to the third axis have a same curvature, and
wherein, in at least one of the plurality of third stacked bodies, an optical axis of the third light control part is non-parallel to the third axis.

9. The light source module according to claim 8, wherein, in at least one of the plurality of third stacked bodies, the third axis is separate from a center of the light emitting surface of the third light emitting part in the top view.

10. The light source module according to claim 9, wherein, in at least one of the plurality of third stacked bodies, the third axis is located closer to the center region than the center of the light emitting surface of the third light emitting part is to the center region in the top view.

11. The light source module of claim 10, wherein, in each one of the plurality of third stacked bodies:
the optical axis of the third light control part is non-parallel to the third axis; and
the third axis is located closer to the center region than the center of the light emitting surface of the corresponding third light emitting part is to the center region in the top view.

12. The light source module of claim 11, wherein, in each of the plurality of third stacked bodies:
third axes of all of the plurality of third stacked bodies are positioned on a circumference of a second virtual circle in the top view; and
the third axis and the center of the light emitting surface of the corresponding third light emitting part are positioned, in the top view, on a second virtual line that passes through a center of the second virtual circle.

13. The light source module according to claim 12, wherein, in each one of the plurality of third stacked bodies, both sides of the third emission surface with respect to the third axis have a same curvature, in a cross section including the second virtual line.

14. The light source module according to one of claim 1 to claim 13, wherein ultraviolet light exits the optical member.

15. An ultraviolet light fluid processing device comprising:
a flow pipe in which a fluid flows; and
the light source module of claim 14, configured to irradiate the ultraviolet light emitted from the optical member onto inside of the flow pipe.
